# EUROPEAN PATENT SPECIFICATION

(11) **EP 2 624 806 B1**
(45) Date of publication and mention of the grant of the patent: **21.06.2017**
(21) Application number: 11781877.3
(22) Date of filing: 03.10.2011
(51) Int. Cl.: A61K 8/19, A61Q 19/08, A61K 8/02

(54) **COSMETIC SKIN CARE COMPOSITIONS COMPRISING INSOLUBLE COPPER OXIDE**
KOSMETISCHE HAUTPFLEGEZUSAMMENSETZUNGEN MIT EINEM UNLÖSLICHEN KUPFEROXID
COMPOSITIONS COSMETIQUES DE SOIN DE LA PEAU

(30) Priority: 04.10.2010 US 389290 P
(43) Date of publication of application: 14.08.2013
(73) Proprietor: Cupron Inc., Richmond, Virginia 23219 (US)
(72) Inventor: GABBAY, Jeffrey, S., Jerusalem 92142 (IL)
(74) Representative: Fairbairn, Angus Chisholm
(86) International application number: PCT/IL2011/000769
(87) International publication number: WO 2012/046229

(56) References cited:
- WO-A2-2007/046083
- WO-A2-2011/051948
- FR-A1- 2 692 482
- FR-A1- 2 901 140
- BORKOW G ET AL: "Improvement of facial skin characteristics using copper oxide containing pillowcases: a double-blind, placebo-controlled, parallel, randomized study", INTERNATIONAL JOURNAL OF COSMETIC SCIENCE, KLUWER ACADEMIC PUBLISHERS, DORDRECHT, NL, vol. 31, no. 6, 1 December 2009 (2009-12-01), pages 437-443, XP009146615, ISSN: 0142-5463, DOI: 10.1111/J.1468-2494.2009.00515.X
- GORTER RW AND AL.: "Examination of the cutaneous absorption of copper after the use of copper-containing ointments.", AMERICAN JOURNAL OF THERAPEUTICS, vol. 11, no. 6, 2004, pages 453-458, XP009167412,
- V. B. PATRAVALE ET AL: "Novel cosmetic delivery systems: an application update", INTERNATIONAL JOURNAL OF COSMETIC SCIENCE, vol. 30, no. 1, 1 February 2008 (2008-02-01), pages 19-33, XP055036522, ISSN: 0142-5463, DOI: 10.1111/j.1468-2494.2008.00416.x
- RABE ET AL: "Photoaging: Mechanisms and repair", JOURNAL OF THE AMERICAN ACADEMY OF DERMATOLOGY, C.V. MOSBY, ST. LOUIS, MO, US, vol. 55, no. 1, 1 July 2006 (2006-07-01), pages 1-19, XP005513432, ISSN: 0190-9622, DOI: 10.1016/J.JAAD.2005.05.010
- GIBBS B F ET AL: "ENCAPSULATION IN THE FOOD INDUSTRY: A REVIEW", INTERNATIONAL JOURNAL OF FOOD SCIENCES AND NUTRITION, CARFAX PUBLISHING LTD, GB, vol. 50, no. 3, 1 January 1999 (1999-01-01), pages 213-224, XP009013399, ISSN: 0963-7486, DOI: 10.1080/096374899101256
- MULLER R H ET AL: "Challenges and solutions for the delivery of biotech drugs - a review of drug nanocrystal technology and lipid nanoparticles", JOURNAL OF BIOTECHNOLOGY, ELSEVIER SCIENCE PUBLISHERS, AMSTERDAM, NL, vol. 113, no. 1-3, 30 September 2004 (2004-09-30), pages 151-170, XP004569606, ISSN: 0168-1656, DOI: 10.1016/J.JBIOTEC.2004.06.007

## Description

### FIELD OF THE INVENTION

The invention relates to a non-therapeutic topical application method for the prevention, mitigation or abrogation of skin aging or skin imperfections in a subject.

### BACKGROUND OF THE INVENTION

Skin exposure to sunlight, pollution and other toxic effects either occasionally or to multiple and/or extreme exposures results in damage thereto. Ultraviolet radiation, environmental pollution and atmospheric pollution, wind, heat, low relative humidity levels, contact with household surfactants and other chemicals, abrasives, smoking, alcohol, drugs, diet, stress, mechanical stress, severe atmospheric conditions and so on impact the appearance and health of the skin, with increasing exposure resulting often in increased skin damage. Other factors, such as aging and the other biochemical changes in the skin, hormonal upheavals, fatigue, acne, obesity, tanning, diets, disease, hematomas and disorders of the blood microcirculation will also deleteriously impact the skin. Resulting effects may include cosmetically undesirable impairment of the visible appearance, clinical and physical properties and physiological and histological functions of the skin and even give rise to visible signs of skin aging.

Some examples of such deleterious effects on the skin include dryness and the development of fine lines and wrinkles, loss of elasticity, wasting and sagging of the skin, loss of firmness, thinning of the skin, loss of uniformity of the complexion, a dull complexion, hyperpigmentation, senile lentigines, red spots, a rough, coarse surface texture and a marbled pigmentation.

For many people, an impaired or aged skin reminds them of the disappearance of youth. For that reason, our societies attribute great importance to apparent youth and fighting against deteriorated/damaged/imperfect skin has become an economic issue. The treatments proposed range from functional cosmetic creams to cosmetic surgery.

There remains a need, therefore, for an inexpensive, safe and reliable method/product for enhancing the morphology, tone, texture and/or appearance of the skin of a subject.

### SUMMARY OF THE INVENTION

This invention provides a non-therapeutic method for the prevention, mitigation or abrogation of skin aging or skin imperfections in a subject, said method comprising topically contacting affected skin in a subject with a composition comprising an insoluble copper oxide as a primary active ingredient therein, wherein said insoluble copper oxide is present at a concentration of between about 0.15 % - about 0.75% w/w in said composition, wherein said composition is a suspension comprising copper oxide particles of less than 10 microns in size, and wherein said copper oxide is encapsulated within a shell of cellulose.

### BRIEF DESCRIPTION OF THE DRAWINGS

Figure 1 is a photograph of a subject treated with the copper-containing formulation, showing front (Panels A and D) and side views (Panels B, C, E and F) at baseline (Panels A, B and C) and following four weeks of the treatment regimen, respectively (Panels D, E and F).
Figure 2 is a photograph of another representative subject treated with the copper-containing formulation, showing front (Panels A and D) and side views (Panels B, C, E and F) at baseline (Panels A, B and C) and following four weeks of the treatment regimen, respectively (Panels D, E and F).
Figure 3 is a photograph of another representative subject treated with the copper-containing formulation, showing front (Panels A and D) and side views (Panels B, C, E and F) at baseline (Panels A, B and C) and following four weeks of the treatment regimen, respectively (Panels D, E and F).
Figure 4 is a photograph of another representative subject treated with the copper-containing formulation, showing front (Panels A and D) and side views (Panels B, C, E and F) at baseline (Panels A, B and C) and following four weeks of the treatment regimen, respectively (Panels D, E and F).

### DETAILED DESCRIPTION OF THE INVENTION

The present invention relates to non-therapeutic methods for the prevention, mitigation or abrogation of skin aging or skin imperfections in a subject.

In embodiments, the present invention uses compositions comprising an effective amount of an insoluble copper oxide as a primary active ingredient therein and either present at a concentration of between about 0.25 % - about 0.75% w/w, or present as particles ranging in a size of from between about 0.5 to about 10 microns.

While compositions comprising an insoluble copper oxide as active ingredient therein have been described to be potent wound healing agents, to date, there has been no positive effect specifically described for prevention, mitigation or abrogation of skin aging or skin imperfections in a subject.

Surprisingly, the compositions used in this invention, when comprising an insoluble copper oxide as a primary active ingredient therein and either present at a concentration of between about 0.15 % - about 0.75% w/w, or present as particles ranging in a size of from between about 0.5 to about 10 microns yield superior results in terms of their positive effect on the appearance of skin, skin aging and skin imperfections. In some aspects of the invention, compositions comprising an insoluble copper oxide as a primary active ingredient therein and either present at a concentration of between about 0.15 % - about 0.5% w/w, or present at a concentration of between about 0.20 % - about 0.25% w/w yielded the most superior results in terms of their positive effect on the appearance of skin, skin aging and skin imperfections. The specifically described low concentration range and particle size range is uniquely beneficial, in comparison to compositions comprising other particle size ranges, and is at least as beneficial, if not more beneficial as a composition comprising a higher weight percentage component of the insoluble copper oxide.

In some embodiments of the invention, the compositions comprise an insoluble copper oxide as a primary active ingredient therein present at a concentration of between about 0.15 % - about 0.75% w/w, and the insoluble copper oxide is present as particles ranging in a size of from between about 0.5 to about 10 microns, and in some embodiments, the composition has a particle size distribution, wherein d₅₀ is less than or equal to 10 microns.

In one embodiment, the insoluble copper oxide particle-containing composition has a particle size distribution, based on cumulative volume, of d 50 less than or equal to 10 µm, such as about 0.5 to 8 µm, or about 2 to 6 µm, and d 50 less than or equal to 10 µm, and in some embodiments less than or equal to about 8 µm. In another preferred embodiment the insoluble copper oxide particle-containing composition has a d 90 less than 6 µm. The terms "d 50" and "d 90" are well understood in the art. For example, a d 90 of 8 µm means that 90% (by volume) of the particles have a size less than or equal to 8 microns; a d 50 of 6 µm means that 50% (by volume) of the particles have a size less than or equal to 6 microns, as tested by any conventionally accepted method such as the laser diffraction method, d 50 and d 90 values can be determined by various techniques known in the art, such as laser diffraction. Suitable methods for laser diffraction, for example, are well known and can be obtained from various sources.

Thus, the pharmaceutical compositions used in this invention can be used to prevent, mitigate or abrogate skin aging or skin imperfections both men and women.

In one embodiment, the reference to prevention, mitigation or abrogation of skin aging or skin imperfections in a subject refers to a qualitative difference in the appearance or quality of the skin in terms of exhibiting signs of imperfection or aging. In one embodiment, the reference to prevention, mitigation or abrogation of skin aging or skin imperfections in a subject refers to a quantitative difference in the appearance or quality of the skin in terms of exhibiting signs of imperfection or aging.

In one embodiment, the reference to prevention refers to reduction of the incidence of the appearance of skin aging or skin imperfections in a subject, when for example, viewed in an overall population, over time.

In one embodiment, the reference to mitigation of skin aging or skin imperfections in a subject refers to a qualitative or quantitative reduction in the appearance of signs of skin aging or skin imperfections in a subject over time.

In one embodiment, the reference to abrogation of skin aging or skin imperfections in a subject refers to the loss of signs of skin aging or skin imperfections in a subject over time.

A composition as described herein for the prevention, mitigation or abrogation of skin aging or skin imperfections in a subject through the topical application of the various formulations designed for topical application as described herein, is optionally combined with at least a second skin therapeutic or stimulatory agent comprising lipoxygenase inhibitors, bradykinin inhibitors, prostaglandins and their derivatives, prostaglandin receptor agonists or antagonists, non- prostanoic prostaglandin analogues, vasodilators, cyclosporins and their analogues, antimicrobials, anti- inflammatories, retinoids, 2 0 benzaikonium chloride, benzethonium chloride, phenol, oestradiol, chlorpheniramine maleate, chlorophyllin derivatives, cholesterol, cysteine, methionine, menthol, peppermint oil, calcium pantothenate, panthenol, resorcinol, protein kinase C activators, 2 5 glycosidase inhibitors, glycosaminoglycanase inhibitors, pyroglutamic acid esters, hexosaccharidic acid or acylhexosaccharic acid, aryl- substituted 6 ethylenes, N-acylated amino acids, flavonoids, ascomycin derivatives and analogues, histamine antagonists, saponins, proteoglycanase inhibitors, oestrogen agonists and antagonists, pseudopterins, cytokines and growth factor promoters, IL-1 or IL-6 inhibitors, IL-10 promoters, TNF inhibitors, vitamins, benzophenones, hydantoin, octopirox, retinoic acid, antipruritic agents, agents for combating parasites, antifungals, nicotinic acid esters, calcium antagonist agents, hormones, triterpenes, anti-androgen agents, steroidal or non-steroidal inhibitors of 5-reductases, potassium channel agonists, FP receptor agonists, or their mixtures.

The compositions used in the present invention may be formulated into a lotion, an ointment, a cream, a patch, a spray, a powder, a sachet, a suspension, an emulsion, a solution, or an oil.

Particularly, the composition used in the present invention may be used by directly spreading or spraying it. Skin surfaces to which the composition is applied include any region of the face, as well as any regions of body, for example, on the neck of the subject. Therefore, the composition may be used as a lotion, cream, spray, mousse, gel, etc.

A dosage of copper oxide used according to the present invention should be appropriately determined considering sex, age, condition of the skin, etc. Generally, a daily dosage for an adult is about .01 to about 1 mg/cm², which may be applied 1 to 5 times a day. In some embodiments, the dosage is a small dab of cream, once every 6 to 12 hours, or in some embodiments, such dosage will be adjusted, as will be appreciated by the skilled artisan, to suit the subject being treated, e.g. some subjects may apply less cream or apply the cream less frequently, whereas other subjects may apply more cream, more frequently.

As described herein, in some embodiments, the invention used a composition formulated for topical application comprising an insoluble copper oxide as active ingredient therein. In some embodiments, the copper oxide is cupric oxide and in some embodiments, the copper oxide is cuprous oxide and in some embodiments, the composition may comprise both cupric and cuprous oxide.

The composition is a suspension comprising copper oxide particles of less than 10 microns in size and in some embodiments, the particles have a size between about 0.5 to about 10 microns.

In other embodiments of the invention, the compositions used will further comprise a suitable carrier, which carrier, in some embodiments, is adapted for topical administration to a subject. In another embodiment, the suitable carrier may comprise of at least one emollient. In further embodiments, the suitable carrier may be a skin penetrating carrier. In preferred embodiments, the skin penetrating carrier may be DMSO, liposomes, lipophilic solvents, lecithin, transcutol, melatonin, nanospheres, nanoshells, cerasomes, and/or rovisomes.

In other embodiments, the skin-penetrating carrier comprises a hollow and solid lipid structure. In another embodiment, the skin-penetrating carrier comprises a nano- structure.

In further embodiments, the suitable carrier is selected from the group consisting of a transdermal patch, lotion, ointment, paste, foam, emulsion, cream, serum, aerosol, spray, roll-on formulation, masque, cleanser, shampoo, conditioner, gel, oil or moisturizer,. In additional embodiments, the suitable carrier can be a lubricating formulation, water- based formulation, silicone-based formulation, petroleum- based formulation, natural-oil based formulation, and/or massage formulation.

In other embodiments of the present invention, the composition used further comprises at least one additional agent. In some embodiments, the said at least one additional agent can be selected from the group consisting of minerals, antimicrobial agents, antioxidants, antigens, analgesics, anti-inflammatory agents, sebum-reducing agents, hormones, enzymes, peptides, proteins, lipids, retinoids, vitamins, wound recovery agents, botanical extracts, MMP inhibitors, integument and skin-supporting components, or massage oils.

The compositions used in the present invention can comprise (open ended) or consist essentially of the components used in the present invention as well as other ingredients described herein. The term, "consisting essentially of" refers herein to the fact that the composition or component may include additional ingredients, but only if the additional ingredients do not materially alter the basic and novel characteristics of the claimed methods. In some embodiments, when the composition consists essentially of an insoluble copper oxide present as particles ranging in a size of from between about 0.5 to about 10 microns, while other therapeutic agents may be included in the composition, the agent primarily responsible for the improved effect on the skin is the incorporation of insoluble copper oxide, at the concentration or particle size noted. Such compositions may further include other additives, and it may be possible to include up to about 10% by weight of materials that could materially alter the basic and novel characteristics of the invention as long as the utility of the compounds (as opposed to the degree of utility) is maintained.

In some embodiments, the composition may further comprise the incorporation of other additives, such as other metal compounds, however, it will be apparent to the skilled artisan that such incorporation is not expected to alter or otherwise materially affect the basic and novel characteristics of the described copper-containing compositions used in this invention.

In one embodiment, where the composition is to be in contact with human or mammalian keratinous tissue, the additional ingredients should be suitable for application to keratinous tissue, that is, when incorporated into the composition they are suitable for use in contact with human keratinous tissue (nails, skin, lips) without undue toxicity, incompatibility, instability, allergic response, and the like within the scope of sound medical judgment. The CTFA Cosmetic Ingredient Handbook, Ninth Edition (2002) describes a wide variety of nonlimiting cosmetic and pharmaceutical ingredients, commonly used in the skin care industry, which are suitable for use as additional ingredients in the compositions used in the present invention. Non-limiting examples of these additional ingredient classes include: healing agents, anti-aging agents, anti-wrinkle agents, moisturizers, antibacterial agents, pesticides, antifongic agents, anti-inflammatory drugs, anti-pruriginous agents, anaesthetic, antiviral agents, keratolytic agents, free radicals scavengers, antiseborrheic, antidandruff agents, anti-acne agents, the agents modulating the differentiation, proliferation or pigmentation of the skin and agents accelerating penetration, cleaning agents, hair conditioning agents, skin conditioning agents, hair styling agents, antidandruff agents, hair growth promoters, Fragrances, sunscreen and/or sunblock compounds, pigments, film formers, hair colors, make-up agents, detergents, pharmaceutical drugs, thickening agents, emulsifiers, humectants, emollients, antiseptic agents, deodorant actives, dermatologically acceptable carriers, surfactants, abrasives, absorbents, aesthetic components such as fragrances, colorings/colorants, essential oils, skin sensates, astringents, etc. (e.g., clove oil, menthol, camphor, eucalyptus oil, eugenol, menthyl lactate, witch hazel distillate), anti-caking agents, antifoaming agents, antimicrobial agents (e.g. iodopropyl butylcarbamate), antioxidants, binders, biological additives, enzymes, enzymatic inhibitors, enzyme-inducing agents, coenzymes, plant extracts, ceramides, peptides, buffering agents, bulking agents, chelating agents, chemical additives, colorants, cosmetic astringents, cosmetic biocides, denaturants, drug astringents, external analgesics, film formers or materials, e.g., polymers, for aiding the film-forming properties and substantivity of the composition (e.g., copolymer of eicosene and vinyl pyrrolidone), quaternary derivatives, agents increasing the substantivity, opacifying agents, pH adjusters, propellants, reducing agents, sequestrants, skin bleaching and lightening agents (e.g., hydroquinone, kojic acid, ascorbic acid, magnesium ascorbyl phosphate, ascorbyl glucosamine), skin-conditioning agents (e.g., humectants, including miscellaneous and occlusive), skin soothing and/or healing agents and derivatives (e.g., panthenol and derivatives (e.g., ethyl panthenol), aloe vera, pantothenic acid and its derivatives, allantoin, bisabolol, and dipotassium glycyrrhizinate), skin treating agents, thickeners, and vitamins and derivatives thereof, and lignans.

The compositions used in the present invention generally contain at least one additional ingredient. The compositions may contain a plurality of additional ingredients as well.

In some embodiments, the composition further comprises an antimicrobial agent selected from the group consisting of triclosan, povidone, iodine, proflavine, honey, hydrogen peroxide, clotrimazole, or sulfur.

In other embodiments, the composition further comprises an antioxidant selected from the group consisting of beta glucan, curcumin, carnosine, polyphenolics, superoxide dismutase (SOD), catalase, glutathione peroxidase, oligomeric proanthocyanidins, bioflavonoids, oligomeric procyanidolic complexes, leuco anthocyanin, anthocyanidin, alpha-lipoic acid, coenzyme Q-10, selenium, vitamin E, vitamin C, lycopene, tocotrienols, or glutathione.

In further embodiments, the composition further comprises an analgesic comprising an amine-containing local anesthetic. In other embodiments, the composition further comprises an antihistamine analgesic. In yet further embodiments, the composition further comprises an analgesic selected from the group consisting of paracetamol, NSAIDs, benzocaine, butamben picrate, dibucaine, dibucaine hydrochloride, dimethisoquin hydrochloride, dyclonine hydrochloride, lidocaine, lidocaine hydrochloride, pramoxine hydrochloride, tetracaine, tetracaine hydrochloride, alcohols and ketones, benzyl alcohol, camphor, combinations of camphor and phenol, camphorated metacresol, juniper tar, menthol, phenol, phenolate sodium, resorcinol, antihistamines, diphenylhydramine hydrochloride, tripelennamine hydrochloride, hydrocortisone preparations, hydrocortisone, hydrocortisone acetate, allyl isothiocyanate, ammonia solutions, methyl salicylate, turpentine oil, histamine dihydrochloride, methyl nicotinate, capsaicin, capsicum, or capsicum oleoresin.

In some embodiments, the composition further comprises anti-inflammatory agents selected from the group consisting of hyssop, licorice extract, aloe, salicylic acid, allantaoin, bisabolol, or fumaric acid.

In other embodiments, the composition further comprises sebum-reducing agent selected from the group consisting of azelaic acid, revivogen, or MK-386 (4,7β-dimethyl-4-aza-5α-cholestan-3-one).

In further embodiments, the composition further comprises a peptide selected from the group consisting of palmitoyl pentapeptide, ubiquitin, oligopeptide, neuropeptide Y, pentapeptide, hexapeptide, acetyl hexapeptide-3, palmitoyl pentapeptide 3, epidermal growth factor (Egf), copper and copper containing peptides, thrombin, or fibroblast growth factor (Fgf).

In other embodiments, the composition further comprises a lipid selected from the group consisting of glycerides, phospholipids, phosphatidylcholine, or lecithin.

Additionally, in some embodiments, the composition further comprises a retinoid selected from the group consisting of tretinoin, retinol, rose hips, or 9-cis retinoic acid.

Additionally, some particular embodiments may further comprise vitamins wherein the said vitamin is selected from the group consisting of vitamin A, B1, B2, B3, B5, B6, B7, B9, B12, C, Ester-C, D, E, F, or K. Other embodiments may comprise vitamin containing compounds such as rose hips.

Further embodiments provide for wound recovery agents selected from the group consisting of allantoin, beta glucan, geranium extract, azelaic acid, curcumin, fumaric acid, gamma linolenic acid, farsenol, or squalene.

In another embodiment, the compositions further comprise MMP inhibitors selected from the group consisting of minimal-domain MMPs, simple hemopexin domain-containing MMPs, gelatin-binding MMPs, furin-activated MMPs, and Vitronectin-like insert MMPs, type I transmembrane MMPs, glycosyl- phosphatidyl inosital (GPI)-linked MMPs, or type II transmembrane MMPs.

In further embodiments, the compositions further comprise polysaccharides such as beta glucan or dextran.

Additionally, some embodiments comprise integument and integument and skin-supporting components selected from the group consisting of vitamin K, borage oil, flax seed, cod liver oil, black currant, alpha and beta hydroxy acids, grape seed, pycnogenol, rose hips, sunscreens, tea tree oil, acetyl glucosamine algae, collagen, elastin, copper PCA, dead sea minerals, glycerin, hormone creams, human growth factor, kinetin, lanolin, mineral oil, olive oil, oxygen, perflurodecalin (Rejuvenox), soy lecithin phospholipids, hydrogen peroxide, triclosan, salicylic acid, papain, aloe vera, lavender oil, geranium oil, chamomile, calendula officinalis, squalane, magnesium oxide crystals, macademia nut oil, galactoarabinan, magnesium aluminum silicate, sweet almond oil, sesame oil, palmitoyl- pentapeptide-3, peptides, benzoic acid, butylene glycol, carbomer, phyllanthus emblica fruit extract, urea, centella asiatica extract, echinacea angustifoila (coneflower) extract, hydrolyzed wheat protein, propylene glycol, bearberry extract, licorice, carnosine, caffeine, cocoa butter, kukui nut, shea butter, mugwort extract (artemesia vulgaris), mango butter, plantago lanceolate leaf extract, xanthan gum, sodium lauryl sulfate, glycolic acid, lactic acid, malic acid, citric acid, aartaric acid, all-trans retinoic acid, allantoin, aloe barbadensis, aminobutyric acid, arbutin, arginine amino acid, azelaic acid, caffeic acid, carnosine, retin- A, ceramides, copper gluconate, superoxide eismutase, curcumin, cystosine, beta glucan, dehydroepiandrosterone, DHEA, dinitrochlorobenzene, dipotassium glycyrrhizinate, hydantoin, DMSO, elastin, Ester-C, bromelian, erythropoietin (EPO), evening primrose oil, farnesol, fumaric acid, GABA (gamma aminobutyric acid/gamma amino-butyric acid), gamma linolenic acid, geranium extract, glutathione, glycyrrhiza glabra glycyrrhetic acid, hesperidin, hyaluronic acid, N6-Furfuryladenine Hormone, kojic acid, Tissue Inhibitors Of Metalloproteinases Matrixyl (TIMPS), vitamin E, vitamin C, flavonoids, palmitoyl panax ginseng root extract, pantothenic acid, pycnogenol scavenger, phosphatidylcholine, resveratrol, retinol, silicone, soy extracts, squalene, sulfur, saw palmetto, tocotrienols, ubiquinone, coenzyme QIO, botox, botulinum toxin, EGF, IGF, calendula officinalis, immortelle, green tea extract, white tea, black tea, glucosamine, algae, yeast, magnesium, magnesium aspartate, glycerin, progesterone, estrogen, lavender, cucumber, DNA, panthenol, zinc gluconate, camellia oleifera leaf extract, or pomegranate.

In additional embodiments, the composition comprises at least one additional agent selected from the group consisting of base components, surfactants, thickening agents, gelling agents, stabilizing agents, emulsifying agents, dispersing agents, suspending agents, humectants, emollients, acidic or alkaline substances, buffering agents, anti-crystalline agents, lubricating agents, coloring agents, perfumes, excipients, foaming agents, diluents, fillers, binding agents, or preservatives.

In another aspect of the present invention, the described components of the composition are encapsulated by a protective membrane. In some embodiments, the protective membrane can be a liposome, nanosphere, rovisome, cerasome, or nanoshell. In another embodiment, the protective membrane encapsulates a portion of the components. In further embodiments, a protective liposomal membrane encapsulates a portion of the components.

The composition pH preferably ranges from about 5 to about 8, more preferably from about 6 to about 7. It can be adjusted by adding acid, such as, for example, hydrochloric acid or citric acid.

The compositions used in the invention may be in all the galenic forms conventionally used for a topical application, including in the form of aqueous, hydroalcoholic or oily solutions, oil-in-water emulsions (O/W), or water-in-oil emulsions (W/O), or multiple emulsions, such as water-in-oil-in-water emulsions (W/O/W) of aqueous or oily gels, of liquid anhydrous products, either pasty or solid, or dispersions of a greasy phase in an aqueous phase in the presence of spherules, such as spherules being adapted to be polymeric nanoparticles such as nanospheres or nanocapsules, or lipidic vesicles, either of a ionic and/or non ionic type. Such compositions are prepared according to the usual methods.

Moreover, compositions used in the invention can be more or less fluid and show the aspect of a white or coloured cream, a balm, a milk, a lotion, a serum, a paste or a foam.

They may possibly be applied onto the skin in the form of an aerosol. They may also be in a solid form, such as a stick.

The compositions used in this invention will, in some embodiments, comprise pharmaceutically acceptable and/or suitable carriers

In some embodiments, the terms, a "pharmaceutically acceptable," "acceptable," and/or "suitable" carrier includes any and/or all solvents, dispersion media, coatings, isotonic, and/or absorption delaying agents and/or the like. The phrases "pharmaceutically," "pharmacologically," "suitable," and/or "acceptable" refer to materials, substances, or compositions that do not produce an adverse, allergic or other untoward reaction when administered to a subject. The selection and use of such materials may be readily determined by one of skill in the art.

Carriers can optionally include one or more components which can be biologically active or inactive. Examples of such optional inactive components include base components (e.g., water, propylene glycol, glycerol, polyethylene glycols, silicones, and/or an oil, such as liquid paraffin, vegetable oil, peanut oil, castor oil, and cocoa butter), surfactants, thickening agents (e.g., aluminum stearate and hydrogen lanolin), gelling agents, stabilizing agents, emulsifying agents, dispersing agents, suspending agents, humectants, emollients, acidic or alkaline substances, buffering agents, anti-crystalline agents, lubricating agents, coloring agents, perfumes, excipients (e.g., starch, tragacanth, and cellulose derivatives), foaming agents, diluents, fillers, binding agents, and preservatives (e.g., methyl paraben, propyl paraben, methylchloroisothiazolinone, and methylisothiazolinone). Suitable carriers also include water-based, silicone-based, petroleum-based, natural-oil based, and massage formulations.

In some embodiments, the carrier may comprise capsules suitable for depositing actives, fragrances, color, glitter etc. onto the skin and integument. Capsules suitable for deposition include, for example, capsules made from mannitol, lactose cellulose, and hydroypropylmethylcellulose. Suitable capsules are, for example, marketed using the Unispheres process of Inducehm, Dubendorf, Switzerland.

The present disclosure also provides for skin-penetrating or skin- permeating carriers. These include, for example, DMSO, liposomes, lipophilic solvents, lecithin, transcutol, nanospheres, nanoshells, and rovisomes.

The insoluble copper oxides of the compositions are encapsulated within a shell of cellulose.

A liposome is a generic term encompassing a variety of single and multilamellar lipid vehicles formed by the generation of enclosed lipid bilayers or aggregates. Liposomes may be characterized as having vesicular structures with a bilayer membrane, generally comprising a phospholipid, and an inner medium that can comprise an aqueous composition.

In some embodiments, some or all of the components of the present invention may be encapsulated in the interior of a liposome, interspersed within the lipid bilayer of a liposome, attached to a liposome via a linking molecule that is associated with the liposome, entrapped in a liposome, complexed with a liposome, etc. The size of a liposome varies depending on the method of synthesis. Liposomes can be a variety of sizes. In preparing such liposomes, any protocol as would be known to one of ordinary skill in the art may be used.

In the context of the present invention, the phrase "Protegenetic liposome" or "Protegenetic liposome complex" generally refers to some embodiments of the present invention, wherein a protective liposomal membrane encapsulates some or all of the components in the skin and integument compositions described.

In further embodiments, more than one protective membrane may be used.

For example, in some embodiments, the composition used in the present invention comprises a first protective membrane encapsulating one or more components of the composition and a second protective membrane encapsulating the first protective membrane and additional components not encapsulated by the first protective membrane. In another embodiment, the composition comprises a first protective membrane encapsulating one or more components of the composition and at least one additional protective membrane encapsulating one or more components of the composition. Regardless of whether a single or multiple protective membranes are used, each individual membrane can be designed to encapsulate a portion of the components of the composition or all components of the composition.

In certain embodiments, the described composition comprises carrier components useful for topical application to skin, integument, and mucosal membrane. This composition can be, for example, a cream, ointment, lotion, cosmetic, soap, wash, facial spray, roll-on formulation, masque, cleanser, moisturizer, and composition-containing pad, patch, strip, or bandage etc. The topical formulation can include other ingredients such as humectants that reduce the rate at which the cream or lotion dries out.

It is known that the cosmetic or dermatological composition used in the invention may also contain usual adjuvants in the cosmetic or dermatological field, such as hydrophilic or lipophilic gelling agents, hydrophilic or lipophilic actives, preservatives, antioxidants, solvents, perfumes, fillers, lipophilic or hydrophilic sun filters, bactericides, odour absorbents, colorants, salts and polymers. The amounts of these various adjuvants are those conventionally used in the involved field and, for example, range from about 0.01 to about 30% of the total weight of the composition. Such adjuvants, according to their nature, can be introduced into the greasy phase, in the aqueous phase and/or in the lipidic spherules.

Fillers adapted to be used in the composition used in the invention may include, for example, besides pigments, fibres; talcum; polyamide particles, more particularly those sold under trademark ORGASOL™ by Atochem company; polyethylene powders; acrylic copolymer based microspheres, such as those made of a copolymer of ethylene glycol dimethacrylate and lauryl methacrylate, sold by Dow Corning company under trademark POLYTRAP™; expanded powders such as hollow microspheres and, more particularly, microspheres sold under trademark EXPANCEL™ by Kemanord Plast company or under trademark MICROPEARL F 80 ED™ by Matsumoto company; powders from natural organic materials such as maize, wheat or rice starches, either cross-linked or not, such as the starch powders cross-linked by the octenylsuccinate anhydride sold under trademark DRY-FLO™ by National Starch company; silicone resin microballs such as those sold under trademark TOSPEARL™ by Toshiba Silicone company; and mixtures thereof. Such fillers may be present in amounts ranging from 0 to 20% wt, and preferably, from 1 to 10% wt based on the total weight of the composition.

Hydrophilic gelling agents may include, for example, carboxyvinyl polymers such as products sold under trademark CARBOPOL™ (CTFA name: carbomer) by Goodrich company, acrylic copolymers such as acrylate/alkylacrylate copolymers, polyacrylamides, and, for example, the mixture sold under tradename SEP1GEL™, 2-acrylamido 2-methylpropane sulfonic acid polymers and copolymers, either cross-linked or not, and neutralized or not, such as the product sold by Hoechst company under trademark "Hostacerin AMPS" (CTFA name: ammonium polyacryl-dimethyltauramide), polysaccharides such as cellulose derivatives, and more particularly hydroxyethylcellulose, natural gums such as xanthane gum, and clays.

Lipophilic gelling agents may include the modified clays such as bentones, fatty acid metal salts, polymers and copolymers of vinyl methyl ether and maleic anhydride such as products sold under trademarks STAB1LEZE™ by ISP company (CTFA name: PVM/MA Decadiene Crosspolymer), and polyethylenes.

Actives may include in particular all the actives known for their activity on skin ageing, such as keratolytic or prodesquamant agents, for example, a-hydroxy-acids, such as glycolic, lactic, malic, citric, tartaric, mandelic acids and the derivatives thereof; β-hydroxy-acids such as salicylic acid and the derivatives thereof; a-aceto-acids such as ascorbic acid or vitamin C and the derivatives thereof; β-ceto-acids; retinoids such as retinol (vitamin A) and the esters thereof (palmitate), retinal, retinoic acid and the derivatives thereof.

Actives may also include vitamins, such as, for example, vitamins B3 or PP (niacinamide), B5 (pantlrnol), E (tocopherol), K1 and the derivatives of those vitamins and more particularly the esters thereof; anti-free radical agents; sun filters; hydrating agents such as polyols; ceramides; and tensing polymers including organics such as latexes, protein hydrolysates and chitin derivatives; DHEA and the derivatives thereof such as alpha-hydroxy-DHEA; Q10 coenzyme, bleaching and depigmenting agents, such as kojic acid para-aminophenol derivatives, arbutin and the derivatives thereof, and the blends thereof.

As chemical sun filters useful in the composition used in the invention, the composition may comprise every UVA and UVB filter useful in the cosmetic field.

UVB filters may include for example: 1. salicylic acid derivatives, more particularly homomenthyl salicylate and octyl salicylate; cinnamic acid derivatives, more particularly 2-ethylhexyl p-methoxycinnamate sold by Givaudan company under trademark PARSOL MCX™; liquid (β'-β-diphenylacrylate) derivatives, more particularly 2-ethylhexyl α-cyano-a-β-diphenylacrylate or octocrylene sold by BASF company under trademark UVINUL N539™; p-aminobenzoic acid derivatives; 4-methyl benzylidene camphor sold by Merck company under trademark EUSOLEX 6300™; 2-phenylbenzimidazole 5-sulfonic acid sold under trademark EUSOLEX 232™ by Merck company; 1,3,5-triazine derivatives, more particularly: 2,4,6-tris[p-2'-ethylhexyl-1'-oxycarbonyl)anilino]-1,3,5-triazine sold by BASF company under trademark UVINUL T150™, and dioctyl butamido triazone sold by Sigma 3V company under trademark UVASORB HEB™, and mixtures of such filters.

UVA filters may include for example: dibenzoylmethane derivatives, more particularly 4-(tert-butyl)4'-methoxy dibenzoylmethane sold by Givaudan company under trademark PARSOL 1789™; benzene 1,4[di(3-methylidenecampho-10-sulfonic)] acid, possibly under a partially or totally neutralized form, sold under trademark MEXORYL SX™ by Chimex company; benzophenone derivatives, for example: 2,4 dihydroxybenzophenone (benzophenone-1), 2,2',4,4'-tetra-hydroxybenzophenone (benzophenone-2), 2-hydroxy-4-methoxy-benzophenone (benzophenone-3) sold under trademark UVINUL M40™ by BASF company, 2-hydroxy-4-methoxy-benzophenone-5-sulfonic acid (benzophenone-4) as well as the sulfonate form thereof (benzophenone-5) sold by BASF company under trademark UVINUL MS40™, 2,2'-dihydroxy-4,4'-dimethoxybenzophenone (benzophenone-6), 5-chloro-2-hydroxybenzophenone (benzophenone-7), 2,2'-dihydroxy-4-methoxy-benzophenone (benzophenone-8), the disodium salt of the 2,2'-dihydroxy-4,4-dimethoxy-benzophenone-5,5'-disulfonic diacid (benzophenone-9), 2-hydroxy-4-methoxy-4'-methyl-benzophenone (benzoph'none-10), benzophenone-11, 2-hydroxy-4-(octyloxy)enzophenone (benzophenone-12); silane derivatives or polyorganosiloxanes with a benzophenone group; anthranilates, more particularly the menthyl anthranilate sold by Haarman & Reiner company under trademark NEO HELIOPAN MA™; compounds having per molecule at least two benzoazolyl groups or at least one benzodiazolyl group, more particularly the 14-bis-benzimidazolyl-phenylene- 3,3',5,5'-tetrasulfonic acid as well as the salts thereof sold by Haarman & Reimer company; silica-based derivatives of N-substituted benzimidazolyl-benzazoles or benzofuranyl-benzazoles, and in particular: 2-[1-[3-[1,3,3,3-tetramethyl-1-[(trimethylsilyl)oxy]-disiloxanyl]-propyl]-1H-benzimidazol-2-yl]-benzoxazole, 2-[1-[3-[,3,3,3-tetramethyl-1-[(trimethylsilyl)oxy]-disiloxanyl]-propyl]-1H-benzimidazol-2-yl]-benzoxazole, 2-[1-(3-trimethylsilanyl-propyl)-1H-benzimidazol-2-yl]-benzoxazole, 6-methoxyl-1,1'-bis(3-trimethylsilanyl-propyl)-1H, 1'H-[2,2']bibenzimidazolyl-benzoxazole, 2-[1-(3-trimethylsilanyl-propyl)-1H-benzimidazol-2-yl]-benzothiazole, which are described in Patent Application EP-A-1 028 120; triazine derivatives, and more particularly, the 2,4- bis {[4-(2-ethyl-hexyloxy)-2-hydroxy]-phenyl}-6-(4-phenyl)-1,3,5-triazine sold by Ciba Geigy compnay under trademark TINOSORB S™ and the 2,2'-methylenebis-[6-(2H benzotriazol-2-yl)4-(1,1,3,3-tetramethylbutyl)-phenol] sold by Ciba Geigy company under trademark TINOSORB M™; and mixtures thereof.

A blend of several of such filters may also be used, as well as a blend of UVB and UVA filters, and also blends with physical filters.

Physical filters may include titanium (amorphous or crystallized titanium dioxide under the rutile or anatase form), zinc, iron, zirconium, cerium oxides or the blends thereof. Such metal oxides can be in the form of particles having a micrometric or a nanometric size (nano-pigments). In the nano-pigment form, the particle average sizes range, for example, from about 5 to about 100 nm. Nano-pigments are preferably used in the composition used in the invention.

Filters usually used in the composition used in the invention include 2-ethylhexyl p-methoxycinnamate sold under trademark Parsol MCX by Hoffmann-Laroche company, benzene 1,4[di(3-methylidenecaampho-10-sulfonic)] acid sold under trademark MEXORYL SX™ by Chimex company, 2-hydroxy-4-methoxy-benzophenone sold under trademark UVINUL M40™ by BASF company, 2-phenylbenzimidazole-5 sulfonic acid sold under trademark EUSOLEX 232™ by Merck company, and the blends thereof.

The invention allows for skin smoothing and skin wrinkle and fine wrinkle attenuation.

The invention therefore also provides a cosmetic treatment method for tensing and smoothing the skin for immediately attenuating the wrinkles and/or the fine wrinkles, characterized in that a composition such as defined here above is applied onto the skin.

The invention also provides a cosmetic treatment method for a wrinkled skin, wherein a composition such as defined hereinabove is applied onto said wrinkled skin, in an effective amount for attenuating wrinkles or fine wrinkles, or other signs of skin aging..

All terms such as "skin aging," "signs of skin aging," "topical application," and the like are used in the sense in which they are generally and widely used in the art of developing, testing and marketing cosmetic and personal care products. The term "cosmetic composition" or more briefly just "composition" in accordance with the present invention relates to a formulation that can be used for cosmetic purposes or as a basis for delivery of one or more pharmaceutical ingredients. It is also possible that these formulations are used for two or more of these same purposes at one time.

In some embodiments, the compositions used in this invention are formulated as part of a cosmetic product.

"Cosmetics," as used herein, include without limitation, lipstick, mascara, rouge, foundation, blush, eyeliner, lipliner, lip gloss, facial or body powder, sunscreens and blocks, mousse, sprays, whether in the form of creams, lotions, gels, ointments, emulsions, colloids, solutions, suspensions, compacts, solids, pencils, spray-on formulations, brush-on formulations and the like.

The compositions used in this invention are useful in the prevention, mitigation or abrogation of skin aging or skin imperfections in a subject.

"Signs of skin aging or skin imperfections" include, but are not limited to, all outward visibly and tactilely perceptible manifestations as well as any other macro or micro effects due to skin aging. Such signs may be induced or caused by intrinsic factors or extrinsic factors, e.g., chronological aging and/or environmental damage. These signs may result from processes which include, but are not limited to, the development of textural discontinuities such as wrinkles and coarse deep wrinkles, skin lines, crevices, bumps, large pores (e.g., associated with adnexal structures such as sweat gland ducts, sebaceous glands, or hair follicles), or unevenness or roughness, loss of skin elasticity (loss and/or inactivation of functional skin elastin), sagging (including puffiness in the eye area and jowls), loss of skin firmness, loss of skin tightness, loss of skin recoil from deformation, discoloration (including undereye rings), blotching, sallowness, hyperpigmented skin regions such as age spots and freckles, keratoses, abnormal differentiation, hyperkeratinization, elastosis, collagen breakdown, and other histological changes in the stratum corneum, dermis, epidermis, the skin vascular system (e.g., telangiectasia or spider vessels), and underlying tissues, especially those proximate to the skin.

Use in prophylactically regulating a skin condition includes delaying, minimizing and/or preventing visible and/or tactile discontinuities in skin (e.g., texture irregularities in the skin which may be detected visually or by feel), including signs of skin aging.

Use in therapeutically regulating a skin condition includes ameliorating, e.g., diminishing, minimizing and/or effacing, discontinuities in skin, including signs of skin aging. Some of the products produced using the compositions of the present invention and indeed the compositions themselves may be used for prophylactically or therapeutically regulating a skin condition.

Some of the products and compositions used in the present invention are useful for improving skin appearance and/or feel. For example, preferred compositions are useful for improving the state of the skin that is imperfect or subject to aggression by providing an immediate visual improvement in skin appearance following application of the composition to the skin. Generally speaking, compositions which further contain particulate materials will be most useful for providing the immediate visual improvement.

Some of the compositions used in the present invention may also provide additional benefits, including stability, absence of significant (consumer-unacceptable) skin irritation, anti-inflammatory activity and good aesthetics.

In some embodiments, the skin exposure to the formulation is for a period of^time of between at least about 6 - 8 hours in a 24-hour period. In some embodiments, for best results, such exposure is every day. In some embodiments, improvement of the skin's appearance as herein described may be seen in as little as 7 days post initiation of treatment, and in some embodiments, subjects will see improvement in the reduction of crow's feet, crow's feet wrinkles, overall skin glow, and overall reduction of the appearance of skin wrinkles in under 28 days.

### Topical Cream

In one embodiment, the present invention provides for use of a topical cream composition comprising Water, Octyldodecanol. Caprylic/Capric Triglyceride, Dimethicone, Ethyl Macadamiate, Cetyl Esters, Tribehenin, Tribehenin PEG-20 Esters, Dimethyl Lauramine Oleate , Butyrospermum Parkii (Shea Butter), Palmitoyl Oligopeptide (Biopeptide CL™), Polysorbate 80, Cyclomethicone, Cetearyl Octanoate, Propylene Glycol, Soluble Collagen, Algae Extract, Sodium Hyaluronate, Colloidal Gold, Immortelle, Beta Glucan, Colloidal Trace Minerals, Gold Unipheres, Dimethicone PEG-8 Meadowfoamate, Sodium PCA, Hydroxyethyl JUrea, Retinyl Palmitate (Vitamin A Palmitate), Tocopheryl Acetate (Vitamin E Acetate), Panthenol (Vitamin B5), Aloe Barbadensis (Aloe Vera) Leaf Juice, Centella Asiatica (Gotu Kola) Extract, Calendula Officinalis (Marigold) Flower Extract, Glycyrrhiza Glabra (Licorice) Root Extract, Camellia Sinensis (Green Tea) Leaf Extract, Juglans Regia (Walnut) Seed Extract, Rosa Mosqueta (Rose Hip) Oil , Glucosamine HCl, Yeast Extract, Urea, Fragrance, Glyceryl Stearate, Beeswax, Tocopherols, Bisabolol, Xanthan Gum, Ammonium Acryloyldimethyltaurate/VP Copolymer, Sodium Acrylate/Sodium Acryloydimethyl Taurate Copolymer, Ethylhexylglycerin, and Phenoxyethanol.

### Masque

In another embodiment, the present invention provides for use of a masque composition comprising at least one component selected from the group consisting of water, sodium magnesium silicate, glycerin, mica, titanium dioxide, polyquaternium-7, hydroxyethyl urea, polyacrylamide, C13-14 isoparaffin, laureth-7, immortelle essential oil, colloidal gold, dipalmitoyl hydroxyproline, tetrahexyldecyl ascorbate, C12-15 alkyl benzoate, tribehenin, ceramide II, PEG-40 rapeseed sterol, palmitoyl oligopeptide, bismuth oxychloride, magnesium sulfate, calcium carbonate, zinc oxide, propylene glycol, methylparaben, propylparaben, potassium sorbate, and fragrance. Preferably, in some non-limiting embodiments, the masque composition comprises components of a percentage by weight listed below: Water from about QS to 100.00% Sodium Magnesium Silicate from about 2.0 - 10.00% Glycerin from about 1.0 - 10.00% Mica from about 0.05 - 3.000% Titanium Dioxide from about 0.05 - 5.000% Polyquaternium-7 from about 0.05 - 1.000% Hydroxyethyl Urea from about 0.05 - 5.000% Polyacrylamide from about 0.05 - 0.500% C13-14 Isoparaffin from about 0.10 - 1.000% Laureth-7 from about 0.05 - 1.000% Helichrysum Angustifolium (Immoretelle) Extract from about 0.001 - 1.00% Colloidal Gold from about 0.001 - 1.00% Dipalmitoyl Hydroxyproline from about 0.001 - 1.00% Tetrahexyldecyl Ascorbate from about 0.001 - 1.00% C12-15 Alkyl Benzoate from about 0.00 1- 1.00% Tribehenin from about 0.001 - 0.50% Ceramide II from about 0.001 - 0.50% PEG-40 Rapeseed Sterol from about 0.00 1- 0.50% Palmitoyl Oligopeptide from about 0.0001 - 0.5% Bismuth Oxychloride from about 0.05 - 1.000% Magnesium Sulfate from about 0.0001 - 1.0% Calcium Carbonate from about 0.0001 - 1.0% Zinc Oxide from about 0.0001 - 1.0% Propylene Glycol from about 0.0 1- 2.000% Methylparaben from about 0.05 - 0.300% Propylparaben from about 0.05 - 0.300% Potassium Sorbate from about 0.01 - 0.300% Fragrance from about 0.01 - 0.500%

In further non-limiting embodiments, the masque composition comprises components of a percentage by weight listed below: Water A Sodium Magnesium Silicate C Glycerin C Colloidal Gold (24K) D Mica (Gold) E Titanium Dioxide E Polyquaternium-7 E Hydroxyethyl Urea E Polyacrylamide E C13-14 Isoparaffin E Laureth-7 E Helichrysum Angustifolium (Immortelle) Extract E Bismuth Oxychloride E Ricinus Communis (Castor) Seed Oil E Phenoxyethanol E Ethylhexylglycerin E Dipalmitoyl Hydroxyproline F Tetrahexyldecyl Ascorbate F Fragrance F Magnesium Sulfate F Calcium Carbonate F Zinc Oxide F Composition Legend A: from about 30% to about 100% B: from about 10% to less than about 30% C: from about 3% to less than about 10% D: from about 1% to less than about 3% E: from about 0.1 % to less than about 1% F: less than about 0.1 %

In additional embodiments, the masque composition further comprises Protegenetic liposomes. In some embodiments, the Protegenetic liposomes comprise from about 0.01 - 1.0 percent by weight of the composition.

### Serums

In another embodiment, the present invention provides for use of a serum composition comprising at least one component selected from the group consisting of cyclopentasiloxane, dimethicone, vinyl dimethicone copolyol crosspolymer, immortelle essential oil, colloidal gold, gold leaf, dipalmitoyl hydroxyproline, and tetrahexyldecyl ascorbate. Preferably, in some non-limiting embodiments, the serum composition comprises components of a percentage by weight listed below: Cyclopentasiloxane from about QS to 100.00% Dimethicone/Vinyl Dimethicone Crosspolymer from about 0.01 - 50.0000% Helichrysum Angustifolium (Immortelle) Extract from about 0.0001 - 1.000% Colloidal Gold from about 0.0001 - 2.000% Gold leaf from about 0.0001 - 1.000% Dipalmitoyl Hydroxyproline from about 0.0010 - 1.000% Tetrahexyldecyl Ascorbate from about 0.0010 - 1.000%

In further non-limiting embodiments, the serum composition comprises components of a percentage by weight listed below: Cyclopentasiloxane A Dimethicone/Vinyl Dimethicone Crosspolymer C Helichrysum Angustifolium (Immortelle) Extract F Gold Leaf (CI 77480) F Dipalmitoyl Hydroxyproline F Tetrahexyldecy] Ascorbate F Colloidal Gold (24K) F Composition Legend A: from about 30 to about 100% B: from about 10% to less than about 30% C: from about 3% to less than about 10% D: from about 1% to less than about 3% E: from about 0.1 % to less than about 1% F: less than about 0.1 %

In additional embodiments, the serum composition further comprises Protegenetic liposomes. In some embodiments, the Protegenetic liposomes comprise from about 0.01 - 1.0 percent by weight of the composition.

By "administering" or "application," it is meant that a composition is delivered to the subject in such a way that it can achieve the desired purpose. Compositions used in the present invention can be administered to any suitable subject, including animals, humans, and other organisms. In the context of animals, the compositions can also be used for veterinary administration to any suitable animal subject such as, for example, cats, dogs, or horses.

It is to be understood that repeated use of reference characters in the present specification and drawings is intended to represent the same or analogous features of the invention.

It will be understood by those skilled in the art that various changes in form and details may be made therein without departing from the scope of the invention as set forth in the appended claims.

In one embodiment, the term "about" refers to a variance of from 1- 10%, or in another embodiment, 5 - 15%, or in another embodiment, up to 10%, or in another embodiment, up to 25% variance from the indicated values, except where context indicates that the variance should not result in a value exceeding 100%.

In the claims articles such as "a,", "an" and "the" mean one or more than one unless indicated to the contrary or otherwise evident from the context. Claims or descriptions that include "or" or "and/or" between members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention also includes embodiments in which more than one, or all of the group members are present in, employed in, or otherwise relevant to a given product or process. Furthermore, it is to be understood that the invention provides, in various embodiments, all variations, combinations, and permutations in which one or more limitations, elements, clauses, descriptive terms, etc., from one or more of the listed claims is introduced into another claim dependent on the same base claim unless otherwise indicated or unless it would be evident to one of ordinary skill in the art that a contradiction or inconsistency would arise. Where elements are presented as lists, e.g. in Markush group format or the like, it is to be understood that each subgroup of the elements is also disclosed, and any element(s) can be removed from the group. It should it be understood that, in general, where the invention, or aspects of the invention, is/are referred to as comprising particular elements, features, etc., certain embodiments of the invention or aspects of the invention consist, or consist essentially of, such elements, features, etc. For purposes of simplicity those embodiments have not in every case been specifically set forth in haec verba herein. Certain claims are presented in dependent form for the sake of convenience, but Applicant reserves the right to rewrite any dependent claim in independent format to include the elements or limitations of the independent claim and any other claim(s) on which such claim depends, and such rewritten claim is to be considered equivalent in all respects to the dependent claim in whatever form it is in (either amended or unamended) prior to being rewritten in independent format

Although described with specific examples, the present invention provides for some embodiments comprising variations of the components described above.

Accordingly, variations, omissions, substitutions, and changes may be made by those skilled in the art without departing from the present invention.

The following examples describe certain embodiments of the invention and and should not be construed as limiting the scope of what is encompassed by the invention in any way.

### EXAMPLES

### EXAMPLE 1:

### INSOLUBLE COPPER OXIDE CONTAINING CREAM FORMULATIONS

In the design of the insoluble copper oxide containing formulations used in this invention, care is taken so that an ionic reservoir of the copper oxide is maintained.

The means for maintaining such reservoir is by maintaining the insoluble copper oxide in a segregated compartment, via encapsulation of the copper oxide particles.

In an embodiment, the encapsulant can be so constructed so as to release the copper oxide in the presence of a desired physical pressure.

The encapsulation formulations rely on the encapsulation of the copper oxide powder within cellulose. In some embodiments, the cellulose is applied as a coating to the individual particles of the powder while in almost liquid form, and following drying creates a hard shell. When formulated in a water-based cream over time, the cellulose becomes soft and when manually manipulated can result in effective release of the copper oxide particles contained therein during topical application.

In another embodiment, encapsulant release may be a function of time.

Micronized Copper Oxide is formulated into an oil-in-water-based emulsion.

A useful formulation consists of lipids, silicones, derivatives, emulsifiers, preservatives, and the copper oxide. A representative composition is as tabulated below: By Weight Silicones 20 Behenyl Alcohol, diluent 10 Emulsifier 5 Protective Polymer 1 Preservative 1.0 Copper Oxide 3.0 Water QS.

In some embodiments of the invention, the control of the release of ions as well as the retention of the ions is maintained when the physical copper oxide particle is encapsulated. When the release of ions is desired the material encapsulating the copper oxide particle needs to be ruptured so that as much as possible the ions will penetrate the dermal layers to stimulate the necessary biological mechanisms for hair growth.

### EXAMPLE 2:

### INSOLUBLE COPPER OXIDE CONTAINING FORMULATION REDUCTION OF SKIN WRINKLES

### Materials and Methods

Copper oxide was purified to 99.9% and ground via modified vector grinding process to an approximate 1 micron powder size. The modified vector grinding process exposes the powder to very high and very low pressures at very quick intervals, causing the powder to "explode". Care was taken to prevent grinding of the powder so that the size was not lower than 0.5 micron.

The thus ground copper oxide powder was encapsulated in an acrylic/cellulose hard cover resulting in white encapsulated particles, which suspended well within a white cream base. Copper oxide was encapsulated in a hard organic cellulose. The encapsulating particle was around 70 microns in size. The shell of the particle is hard and stays that way until it has been in the cream for around 72 hours by which time it softens and breaks easily {see for example, WO/2009/138978}.

After 72 hours post encapsulation, the encapsulated particles were lightly rubbed between fingers, and release of the encapsulated contents was ascertained.

A cream formulation containing 0.4% insoluble copper oxide was prepared. The commercially available Pond's Face and Hand Cream base composition was used. Encapsulated copper oxide particles prepared as above were mixed into the cream base at room temperature. The amount of active ingredient desired was added to the ready cream just before it was bottled and mixed for no less than 20 minutes in a very simple mixer that can run very slowly if desired. Mixing was conducted for example, for 300 revolutions per minute for 20 minutes mixing 200 ml of cream with 7 grams of powder for a final load of 3% active powder.

### Results:

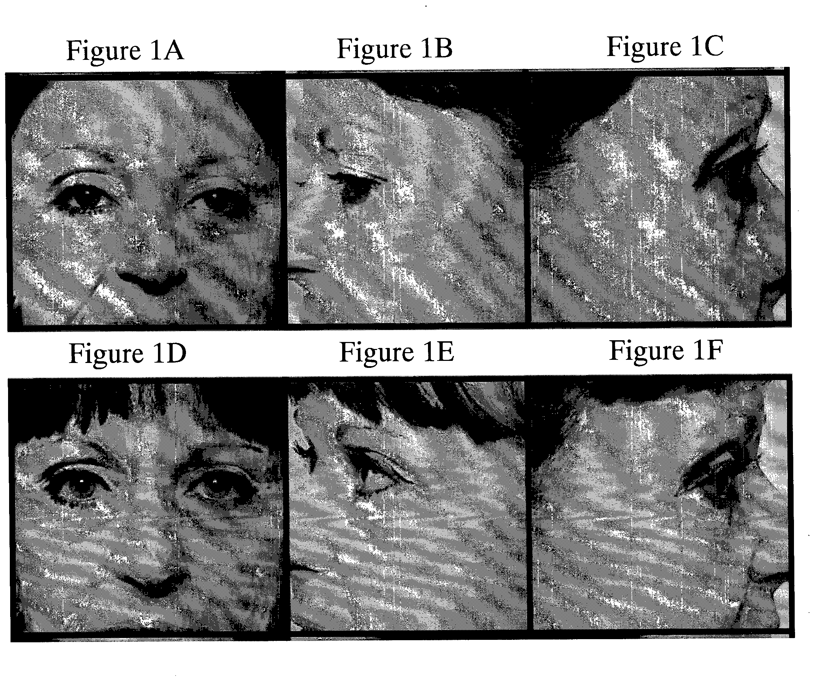

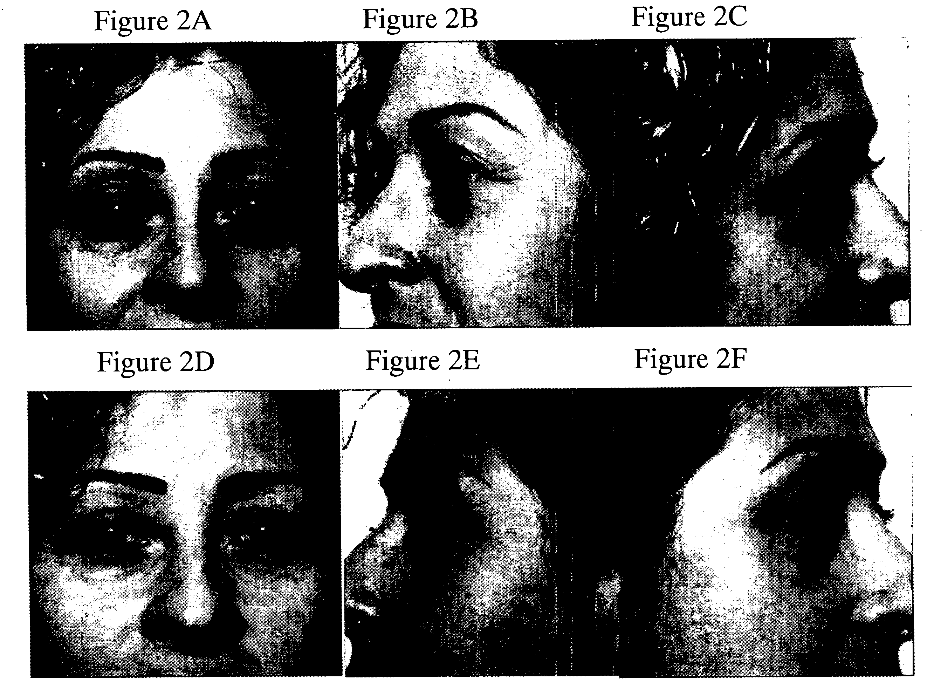

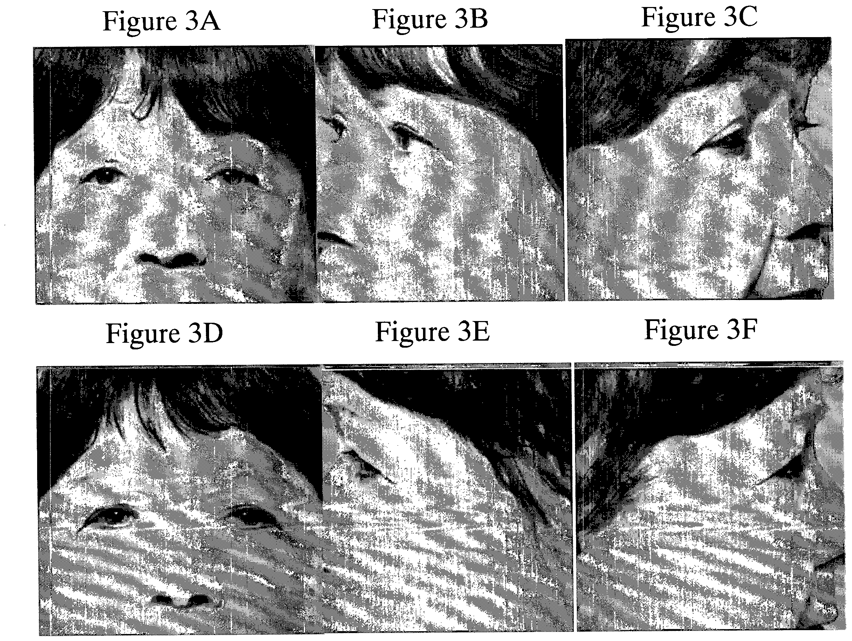

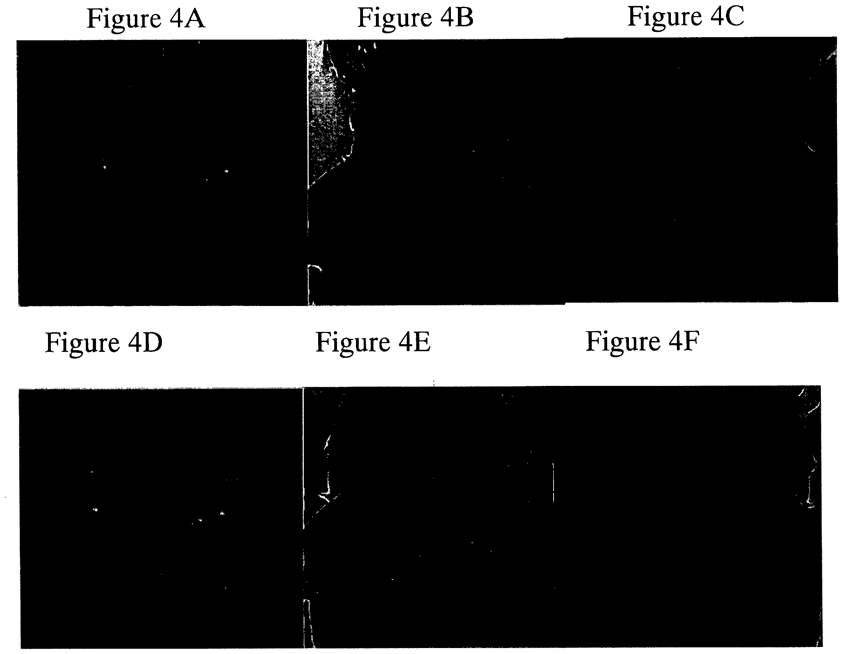

More than 30 subjects were assessed for the effects of the insoluble copper-containing cream composition. Subjects applied a small dab of the copper-containing cream or placebo once a day for 4 weeks in the morning. Participants confirmed compliance. 15 subjects topically applied a placebo formulation containing Ponds base cream alone and more than 15 subjects received the copper-containing formulation. Subjects were photographed at baseline and four weeks after commencing application of the cream. Figures 1-4 A-F depict four subjects treated with the copper-containing formulation, and front and side full face photographs were taken at baseline ("before") and following four weeks of the treatment regimen ("after"). As can be seen from each figure (comparing Panels D-F to A-C, which show front, left and right side views, respectively), there is a reduction in the number of skin wrinkles, and an improvement in the skin quality such that remaining wrinkles appear faint and more superficially located in the skin. Follow up studies indicated that comparable cream formulations containing only 0.25% insoluble copper oxide were as effective and yielded the desirable results showing a reduction in the number of skin wrinkles and improvement in the skin quality (data not shown).

## Claims

1. A non-therapeutic method for the prevention, mitigation or abrogation of skin aging or skin imperfections in a subject, said method comprising topically contacting affected skin in a subject with a composition comprising an insoluble copper oxide as a primary active ingredient therein, wherein said insoluble copper oxide is present at a concentration of between about 0.15 % - about 0.75% w/w in said composition, wherein said composition is a suspension comprising copper oxide particles of less than 10 microns in size, and wherein said copper oxide is encapsulated within a shell of cellulose.

2. The method of claim 1, wherein said composition is a suspension comprising copper oxide particles of between 0.5 to 10 microns in size.

3. The method of claim 1, wherein said insoluble copper oxide is present at a concentration of between about 0.15 % - about 0.5 % w/w.

4. The method of claim 1, wherein said insoluble copper oxide is present at a concentration of between about 0.20 % - about 0.25 % w/w.

5. The method of claim 1, wherein said copper oxide is cupric oxide.

6. The method of claim 1, wherein said copper oxide is cuprous oxide.

## Patentansprüche

1. Nichttherapeutisches Verfahren zur Verhinderung, Milderung oder Abschaffung von Hautalterung oder von Hautunebenheiten bei einem Subjekt, wobei das Verfahren topisches In-Kontakt-Bringen der betroffenen Haut bei einem Subjekt mit einer Zusammensetzung umfasst, die ein unlösliches Kupferoxid als primärer Wirkstoff darin umfasst, wobei das unlösliche Kupferoxid in einer Konzentration zwischen etwa 0,15 % - etwa 0,75 % Gew./Gew. in der Zusammensetzung vorliegt, wobei die Zusammensetzung eine Suspension ist, die Kupferoxidteilchen in einer Größe von weniger als 10 Mikron umfasst, und wobei das Kupferoxid innerhalb eines Mantels von Cellulose eingekapselt ist.

2. Verfahren nach Anspruch 1, wobei die Zusammensetzung eine Suspension ist, die Kupferoxidteilchen einer Größe zwischen 0,5 und 10 Mikron umfasst.

3. Verfahren nach Anspruch 1, wobei das unlösliche Kupferoxid in einer Konzentration zwischen etwa 0,15 % - etwa 0,5 % Gew./Gew. vorliegt.

4. Verfahren nach Anspruch 1, wobei das unlösliche Kupferoxid in einer Konzentration zwischen etwa 0,20 % - etwa 0,25 % Gew./Gew. vorliegt.

5. Verfahren nach Anspruch 1, wobei das Kupferoxid Kupfer(II)oxid ist.

6. Verfahren nach Anspruch 1, wobei das Kupferoxid Kupfer(I)oxid ist.

## Revendications

1. Procédé non thérapeutique de prévention, atténuation ou annulation du vieillissement cutané ou des imperfections cutanées chez un sujet, ledit procédé comprenant la mise en contact de manière topique de la peau concernée chez un sujet avec une composition comprenant un oxyde de cuivre insoluble comme ingrédient actif primaire à l'intérieur, ledit oxyde de cuivre insoluble étant présent sous une concentration comprise entre environ 0,15 % à environ 0,75 % en pds/pds dans ladite composition, ladite composition étant une suspension comprenant des particules d'oxyde de cuivre de moins de 10 microns de taille, et ledit oxyde de cuivre étant encapsulé à l'intérieur d'une enveloppe de cellulose.

2. Procédé selon la revendication 1, ladite composition étant une suspension comprenant des particules d'oxyde de cuivre d'une taille comprise entre 0,5 à 10 microns.

3. Procédé selon la revendication 1, ledit oxyde de cuivre insoluble étant présent sous une concentration comprise entre environ 0,15 % à environ 0,5 % en pds/pds.

4. Procédé selon la revendication 1, ledit oxyde de cuivre insoluble étant présent sous une concentration comprise entre environ 0,20 % à environ 0,25 % en pds/pds.

5. Procédé selon la revendication 1, ledit oxyde de cuivre étant l'oxyde cuprique.

6. Procédé selon la revendication 1, ledit oxyde de cuivre étant l'oxyde cupreux.
